# EUROPEAN PATENT SPECIFICATION

(11) **EP 4 193 963 B1**
(45) Date of publication and mention of the grant of the patent: **19.11.2025**
(21) Application number: 21853784.3
(22) Date of filing: 25.06.2021
(51) Int. Cl.: A61F 2/24, A61M 25/01

(54) **DELIVERY SYSTEM FOR MITRAL VALVE REPAIR**
FREISETZUNGSSYSTEM FÜR MITRALKLAPPENREPARATUR
SYSTÈME DE DISTRIBUTION POUR RÉPARATION DE VALVULE MITRALE

(30) Priority: 04.08.2020 CN 202010769580
(43) Date of publication of application: 14.06.2023
(73) Proprietor: Shanghai Shenqi Medical Technology Co., Ltd., Shanghai 201203 (CN)
(72) Inventor: WANG, Sen, Shanghai 201203 (CN); SONG, Rui, Shanghai 201203 (CN); ZHOU, Guolei, Shanghai 201203 (CN); WANG, Hailan, Shanghai 201203 (CN); DAI, Zhihao, Shanghai 201203 (CN); SU, Yan, Shanghai 201203 (CN)
(74) Representative: Ricker, Mathias
(86) International application number: PCT/CN2021/102292
(87) International publication number: WO 2022/028143

(56) References cited:
- WO-A1-2019/233353
- CN-A- 102 802 561
- CN-A- 107 206 210
- CN-A- 107 233 145
- CN-A- 107 684 657
- CN-A- 109 906 063
- CN-A- 111 012 548
- CN-A- 111 643 228
- CN-U- 202 020 777
- US-B2- 10 369 356

## Description

### TECHNICAL FIELD

The present application relates to the technical field of medical devices, for example, a delivery system for mitral valve repair.

### BACKGROUND

The mitral valve is a valve between the left ventricle and the left atrium. Mitral regurgitation (MR) is the most common heart valve disease caused by poor anastomosis of anterior and posterior mitral leaflets due to organic or functional changes of the mitral valve leaflets, the annulus, the papillary muscle, and the chordae tendineae. According to relevant statistical data, the number of patients with the MR is about 10 times the number of patients with aortic stenosis. There are 4.6 million patients with the MR in the United States, while the number of patients with severe MR in China is up to 10 million.

The MR is now generally treated surgically. Compared with the traditional surgical median sternotomy, the minimally invasive small incision surgery has the advantages of minimal invasion and more safety during the operation. At present, the minimally invasive method mainly includes the transapical technique and the transseptal technique. The transapical technique uses the apical puncture approach to deliver a mitral valve repair device, and the traditional transapical interventional surgery has great invasion.

In contrast to the transapical technique, the transseptal technique uses a transfemoral approach to deliver the mitral valve repair device, and a primary route of the operation is: inferior vena cava-right atrium-interatrial septum-left atrium-mitral valve, which is less invasive and more suitable for the mitral valve treatment. However, since a process of delivering the mitral valve repair device involves a complex spatial angle change, the mitral valve and the surrounding chordae tendineae are very vulnerable, and the existing delivery system is not flexible enough to achieve multi-angle accurate conversion and cannot satisfy requirements of the mitral valve repair device for the multi-angle conversion. In addition, it is relatively difficult to operate and has relatively high requirements for the technical level and clinical experience of the doctor, thus seriously restricting the clinical use of the transseptal technique. Document WO 2019/233353 discloses an implant delivery tube comprises an outer tube and a sheath tube in order from the proximal end to the distal end. The inner cavity of the sheath tube is suitable for receiving the implant. The sheath tube is a telescopic structure, and the outer tube can drive the sheath tube to perform a telescopic motion to change the sheath tube longer or shorter.

### SUMMARY

The present application provides a delivery system for mitral valve repair, which has high flexibility, can achieve multi-angle conversion, and is not easy to shift during delivery, so as to reduce the operation difficulty. The invention is defined by claim 1.

An embodiment provides a delivery system for mitral valve repair. The delivery system for mitral valve repair includes a sheath assembly, a first pull wire, a second pull wire, and a push-pull wire. The sheath assembly includes an outer sheath, a middle sheath, and an inner sheath, where the inner sheath, the middle sheath, and the outer sheath are nested from inside to outside in sequence and slidably connected in sequence along an axial direction of the inner sheath, the outer sheath includes an outer sheath proximal end and an outer sheath distal end connected to each other, hardness of the outer sheath proximal end is greater than hardness of the outer sheath distal end, the outer sheath distal end is capable of being deflected by a first predetermined angle in a first direction relative to the outer sheath proximal end, the middle sheath includes a middle sheath proximal end and a middle sheath distal end connected to each other, hardness of the middle sheath proximal end is greater than hardness of the middle sheath distal end, and the middle sheath distal end is capable of being deflected by a second predetermined angle in a second direction relative to the middle sheath proximal end.

The outer sheath is provided with a first wire lumen along an axial direction of the outer sheath, the first pull wire is disposed in the first wire lumen, a first end of the first pull wire is fixedly connected to the outer sheath distal end, and a second end of the first pull wire is configured to deflect the outer sheath distal end by a first deflection angle in a third direction relative to the outer sheath proximal end when the second end of the first pull wire is pulled.

The middle sheath is provided with a second wire lumen along an axial direction of the middle sheath, the second pull wire is disposed in the second wire lumen, a first end of the second pull wire is fixedly connected to the middle sheath distal end, and a second end of the second pull wire is configured to deflect the middle sheath distal end by a second deflection angle in a fourth direction relative to the middle sheath proximal end when the second end of the second pull wire is pulled.

A third wire lumen penetrates through the inner sheath along the axial direction of the inner sheath, the push-pull wire is disposed in the third wire lumen, a first end of the push-pull wire is connected to a device to be delivered, and a second end of the push-pull wire is configured to be capable of adjusting a position of the device to be delivered when the second end of the push-pull wire is pushed or pulled.

Optionally, the outer sheath proximal end includes a first inner layer, a first braided layer, a reinforcement layer, a second braided layer, and a first outer layer that are nested from inside to outside in sequence, and the first wire lumen penetrates through two ends of the outer sheath proximal end.

The outer sheath distal end includes a second inner layer, a third braided layer, and a second outer layer that are nested from inside to outside in sequence, and the first wire lumen penetrates through an end of the second outer layer facing the outer sheath proximal end.

Optionally, a braid density of the first braided layer is greater than a braid density of the second braided layer.

Optionally, a braid density of the third braided layer is less than a braid density of the second braided layer.

Optionally, the middle sheath proximal end includes a third inner layer and a third outer layer which are nested from inside to outside in sequence, and the second wire lumen penetrates through two ends of the third outer layer.

The middle sheath distal end includes a fourth inner layer, a fourth braided layer, a fourth outer layer, and a fifth outer layer that are nested from inside to outside in sequence, and the second wire lumen penetrates through an end of the fourth outer layer facing the middle sheath proximal end.

Optionally, the inner sheath includes a reinforcement layer, a connecting layer, a fifth braided layer, and a sixth outer layer that are nested from inside to outside in sequence, and the third wire lumen penetrates through two ends of the connecting layer.

Optionally, a reinforcement braided structure is provided in part of the first wire lumen located at the outer sheath distal end.

Optionally, a reinforcement braided structure is provided in part of the second wire lumen located at the middle sheath distal end.

Optionally, a sliding groove is provided on one of an inner wall of the outer sheath and an outer wall of the middle sheath, and a slider is provided on the other one of the inner wall of the outer sheath and the outer wall of the middle sheath, where the slider is slidably connected in the sliding groove.

Optionally, the delivery system for mitral valve repair further includes a metal fixed head.

The metal fixed head is fixedly connected to a distal end of the inner sheath.

Optionally, a lead-in chamfer is circumferentially provided at an end of the metal fixed head facing away from the distal end of the inner sheath, and the lead-in chamfer is at a preset acute angle to an end surface of the metal fixed head.

Optionally, multiple first wire lumens are provided, the multiple first wire lumens are uniformly distributed along a circumferential direction of the outer sheath, and at least one first pull wire is provided in each of the multiple first wire lumens.

Optionally, multiple second wire lumens are provided, the multiple second wire lumens are uniformly distributed along a circumferential direction of the middle sheath, and at least one second pull wire is provided in each of the multiple second wire lumens.

Optionally, multiple third wire lumens are provided, the multiple third wire lumens are uniformly distributed along a circumferential direction of the inner sheath, and at least one push-pull wire is provided in each of the multiple third wire lumens.

### BRIEF DESCRIPTION OF DRAWINGS

FIG. 1 is a structural view of a sheath assembly of a delivery system for mitral valve repair according to the present application;
FIG. 2 is a structural view of an outer sheath of a delivery system for mitral valve repair according to the present application;
FIG. 3 is a structural view of an outer sheath proximal end of a delivery system for mitral valve repair according to the present application;
FIG. 4 is a sectional view of an outer sheath distal end of a delivery system for mitral valve repair according to the present application;
FIG. 5 is a partial sectional view of an outer sheath distal end of a delivery system for mitral valve repair according to the present application;
FIG. 6 is a structural view of a predetermined outer sheath of a delivery system for mitral valve repair according to the present application;
FIG. 7 is a structural view of a middle sheath of a delivery system for mitral valve repair according to the present application;
FIG. 8 is a sectional view of a middle sheath proximal end of a delivery system for mitral valve repair according to the present application;
FIG. 9 is a sectional view of a middle sheath distal end of a delivery system for mitral valve repair according to the present application;
FIG. 10 is a partial sectional view of a middle sheath distal end of a delivery system for mitral valve repair according to the present application;
FIG. 11 is an assembly view of an outer sheath and a middle sheath of a delivery system for mitral valve repair according to the present application;
FIG. 12 is a structural view of an inner sheath of a delivery system for mitral valve repair according to the present application;
FIG. 13 is an assembly view of an inner sheath and a metal fixed head of a delivery system for mitral valve repair according to the present application; and
FIG. 14 is an assembly view of an inner sheath, a metal fixed head, and a device to be delivered of a delivery system for mitral valve repair according to the present application.

### Reference list

- 1: outer sheath
- 11: outer sheath proximal end
- 111: first inner layer
- 112: first braided layer
- 113: reinforcement layer
- 114: second braided layer
- 115: first outer layer
- 116: sliding groove
- 12: outer sheath distal end
- 121: second inner layer
- 122: third braided layer
- 123: second outer layer
- 13: first wire lumen
- 2: middle sheath
- 21: middle sheath proximal end
- 211: third inner layer
- 212: third outer layer
- 22: middle sheath distal end
- 221: fourth inner layer
- 222: fourth braided layer
- 223: fourth outer layer
- 224: fifth outer layer
- 23: slider
- 24: second wire lumen
- 3: inner sheath
- 31: reinforcement layer
- 32: connecting layer
- 33: fifth braided layer
- 34: sixth outer layer
- 35: third wire lumen
- 4: metal fixed head
- 41: lead-in chamfer
- 5: reinforcement braided structure
- 100: device to be delivered

### DETAILED DESCRIPTION

In the description of the present application, it is to be noted that the orientations or position relations indicated by terms such as "center", "above", "below", "left", "right", "vertical", "horizontal", "inside", "outside", and the like are based on orientations or position relations shown in the drawings. These orientations or position relations are intended only to facilitate and simplify the description of the present application and not to indicate or imply that an apparatus or element referred to must have such specific orientations or must be configured or operated in such specific orientations. Thus, these orientations or position relations are not to be construed as limiting the present application. In addition, terms such as "first" and "second" are used only for the purpose of description and are not to be construed as indicating or implying relative importance. Terms "first position" and "second position" are two different positions.

In the description of the present application, it is to be noted that terms such as "mounted", "joined", and "connected" are to be understood in a broad sense unless otherwise expressly specified and limited. For example, the term "connected" may refer to "securely connected" or "detachably connected"; may refer to "mechanically connected" or "electrically connected"; or may refer to "connected directly", "connected indirectly through an intermediary", or "connected in two components". For those of ordinary skill in the art, specific meanings of the preceding terms in the present application may be understood based on specific situations.

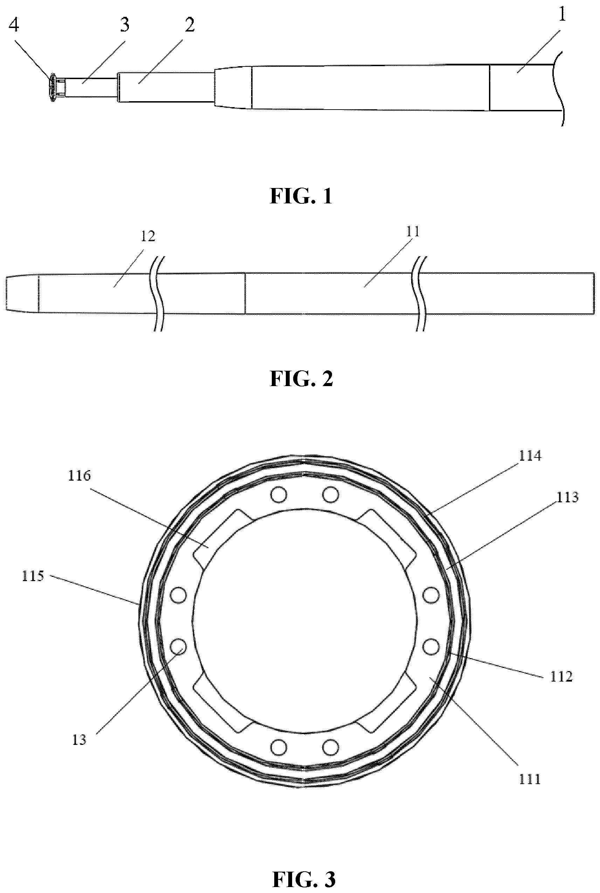

This embodiment provides a delivery system for mitral valve repair. The delivery system can deliver a mitral valve repair device to the mitral valve to cure the MR. As shown in FIGS. 1 to 14, the delivery system for mitral valve repair includes a sheath assembly, a first pull wire, a second pull wire, and a push-pull wire. The sheath assembly is a delivery body of the delivery system and includes an outer sheath 1, a middle sheath 2, and an inner sheath 3, where the inner sheath 3, the middle sheath 2, and the outer sheath 1 are all hollow tubular structures, and the inner sheath 3, the middle sheath 2, and the outer sheath 1 are nested from inside to outside in sequence and slidably connected in sequence along an axial direction of the inner sheath 3. The middle sheath 2 moves relative to the outer sheath 1, and the middle sheath 2 moves relative to the inner sheath 3 so that a length of the entire sheath assembly can be changed, and a device to be delivered 100 connected to the inner sheath 3 is delivered to the mitral valve. The device to be delivered 100 here refers to the mitral valve repair device.

The outer sheath 1 is provided with a first wire lumen 13 along an axial direction of the outer sheath 1, the first pull wire is disposed in the first wire lumen 13, a first end of the first pull wire is fixedly connected to an outer sheath distal end 12, and a second end of the first pull wire is configured to deflect the outer sheath distal end 12 by a first deflection angle in a third direction relative to an outer sheath proximal end 11 when the second end of the first pull wire is pulled. The middle sheath 2 is provided with a second wire lumen 24 along an axial direction of the inner sheath 2, the second pull wire is disposed in the second wire lumen 24, a first end of the second pull wire is fixedly connected to an inner sheath distal end 22, and a second end of the second pull wire is configured to deflect the middle sheath distal end 22 by a second deflection angle in a fourth direction relative to an middle sheath proximal end 21 when the second end of the second pull wire is pulled. A third wire lumen 35 penetrates through the inner sheath 3 along the axial direction of the inner sheath 3, the push-pull wire is disposed in the third wire lumen 35, a first end of the push-pull wire is connected to the device to be delivered 100, and a second end of the push-pull wire is configured to be capable of adjusting a position of the device to be delivered 100 when the second end of the push-pull wire is pushed or pulled.

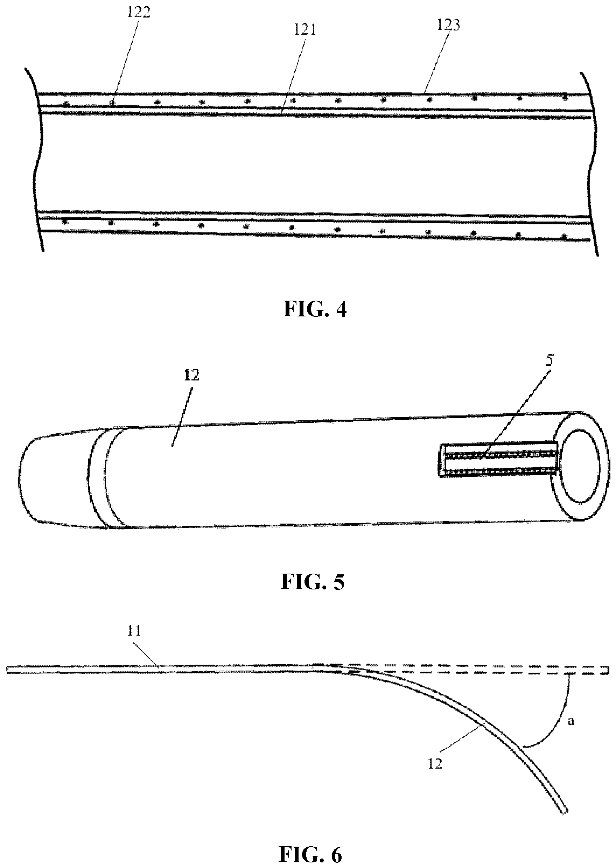

As shown in FIG. 2, the outer sheath 1 includes the outer sheath proximal end 11 and the outer sheath distal end 12 connected to each other. To reduce the operation difficulty of the deflection of the outer sheath 1 to facilitate the operation of a doctor during the operation, as shown in FIG. 6, the outer sheath distal end 12 is deflected by a first predetermined angle a in a first direction relative to the outer sheath proximal end 11. Optionally, the first predetermined angle a is 30° to 60°, and the first direction here is set according to requirements and not specifically limited. Optionally, the outer sheath proximal end 11 and the outer sheath distal end 12 are different in both structure and hardness. In this embodiment, hardness of the outer sheath proximal end 11 is greater than hardness of the outer sheath distal end 12, thereby facilitating pushing.

Optionally, as shown in FIG. 3, the outer sheath proximal end 11 includes a first inner layer 111, a first braided layer 112, a reinforcement layer 113, a second braided layer 114, and a first outer layer 115 that are nested from inside to outside in sequence. The first inner layer 111, the reinforcement layer 113, and the first outer layer 115 may all be made of medical polymer materials such as poly tetra fluoroethylene (PTFE), polyamide (PA), polyether copolyamide (pebax), thermoplastic polyurethanes (TPU), polyethylene (PE), or a blend thereof. The first braided layer 112 and the second braided layer 114 may each be a multi-strand braided structure. Optionally, 8 to 32 braided strands may be provided. Optionally, a braid density PPI of the second braided layer 114 is less than a braid density PPI of the first braided layer 112, so as to improve the overall compression and torsion resistance of the outer sheath proximal end 11. In addition to the multi-strand braided structure, the first braided layer 112 and the second braided layer 114 may each be a single-strand or multi-strand spring braided structure, and a braided wire may be a metal flat wire or a metal round wire with a diameter of 0.02 mm to 0.4 mm. Optionally, a pitch of the second braided layer 114 is greater than a pitch of the first braided layer 112.

As shown in FIG. 4, the outer sheath distal end 12 includes a second inner layer 121, a third braided layer 122, and a second outer layer 123 that are nested from inside to outside in sequence. The second inner layer 121 and the second outer layer 123 may both be made of medical polymer materials such as PTFE, PA, PEBAX, TPU, PE, or a blend thereof. The third braided layer 122 may be a multi-strand braided structure. Optionally, 8 to 32 braided strands may be provided. Optionally, a braid density PPI of the third braided layer 122 is less than the braid density PPI of the first braided layer 112 and the braid density PPI of the second braided layer 114. In addition to the multi-strand braided structure, the third braided layer 122 may be a single-strand or multi-strand spring braided structure, and a braided wire may be a metal flat wire or a metal round wire with a diameter of 0.02 mm to 0.4 mm. Optionally, a pitch of the third braided layer 122 is greater than the pitch of the first braided layer 112 and the pitch of the second braided layer 114.

The outer sheath proximal end 11 and the outer sheath distal end 12 are configured to be different multi-layer structures so that a reasonable balance of flexibility and hardness of the outer sheath 1 can be achieved, thereby avoiding that the outer sheath distal end 12 is so flexible that the outer sheath distal end 12 ruptures during use, avoiding that the outer sheath distal end 12 is too stiff to achieve desired deflection or easy delivery, and providing the outer sheath proximal end 11 with reasonable hardness for ease of handling by the doctor.

In this embodiment, the first inner layer 111 of the outer sheath proximal end 11 is the thickest, and the first wire lumen 13 penetrates through two ends of the first inner layer 111 of the outer sheath proximal end 11 along the axial direction of the outer sheath 1. In addition, the first wire lumen 13 continues to extend in the second outer layer 123 of the outer sheath distal end 12 after penetrating through the outer sheath proximal end 11, so as to form a blind hole structure at the outer sheath distal end 12. The first end of the first pull wire is fixed at the bottom of the blind hole structure, and the second end of the first pull wire protrudes out of the outer sheath proximal end 11. The outer sheath distal end 12 can be deflected by the first deflection angle in the third direction relative to the outer sheath proximal end 11 by pulling the first pull wire. The third direction here may be the same as or different from the first direction and may be adjusted according to requirements. Optionally, the material of the first pull wire may be stainless steel, the nickel titanium alloy, the elgiloy alloy, the cobalt chromium alloy, or the like.

Optionally, as shown in FIG. 5, a reinforcement braided structure 5 is provided inside the first wire lumen 13 at the outer sheath distal end 12, so as to prevent the outer sheath distal end 12 from being too flexible and thus prevent the first pull wire from breaking out of the second outer layer 123 of the outer sheath distal end 12 after being deflected to a desired angle, thereby enhancing surgical safety. In this embodiment, the reinforcement braided structure 5 is a spring tube, and the material of the spring tube may be a metal, such as stainless steel and the platinum tungsten alloy, or may be a polymer material, such as PA and high density polyethylene (HDPE).

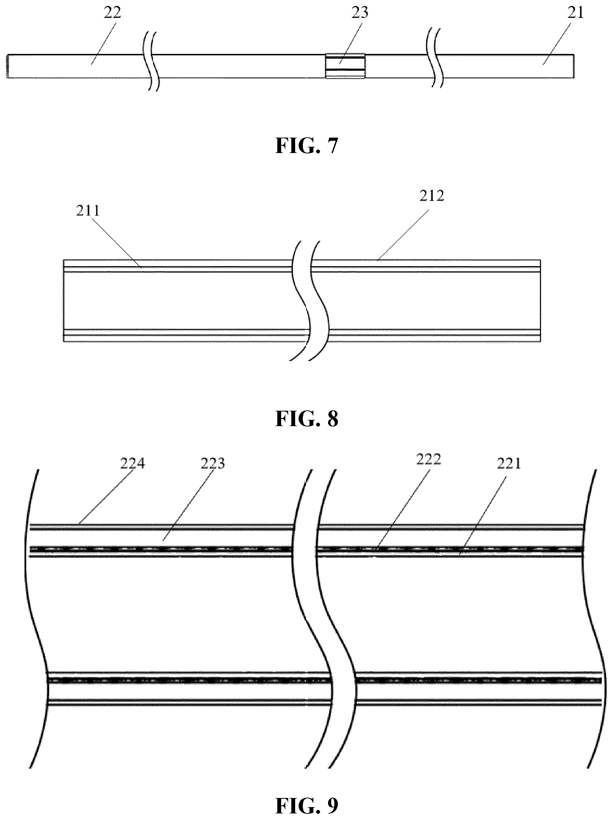

As shown in FIG. 7, the middle sheath 2 includes a middle sheath proximal end 21 and a middle sheath distal end 22 connected to each other. To reduce the operation difficulty of the deflection of the middle sheath 2 to facilitate the operation of the doctor during the operation, the middle sheath distal end 22 is deflected by a second predetermined angle in a second direction relative to the middle sheath proximal end 21. Optionally, the second predetermined angle is 90° to 160°. The second direction here may be the same as or different from the first direction and the third direction and may be selected according to requirements. Hardness of the middle sheath proximal end 21 is greater than the middle sheath distal end 22, facilitating pushing.

Optionally, as shown in FIG. 8, the middle sheath proximal end 21 includes a third inner layer 211 and a third outer layer 212 that are nested from inside to outside in sequence. The third inner layer 211 and the third outer layer 212 may both be made of medical polymer materials such as PTFE, PA, PEBAX, TPU, PE, or a blend thereof. As shown in FIG. 9, the middle sheath distal end 22 includes a fourth inner layer 221, a fourth braided layer 222, a fourth outer layer 223, and a fifth outer layer 224 that are nested from inside to outside in sequence. The fourth inner layer 221, the fourth outer layer 223, and the fifth outer layer 224 may all be made of medical polymer materials such as PTFE, PA, PEBAX, TPU, PE, or a blend thereof. The fourth braided layer 222 may be a multi-strand braided structure. Optionally, 8 to 32 braided strands may be provided. In addition to the multi-strand braided structure, the fourth braided layer 222 may be a single-strand or multi-strand spring braided structure, and a braided wire may be a metal flat wire or a metal round wire with a diameter of 0.02 mm to 0.4 mm. The middle sheath proximal end 21 and the middle sheath distal end 22 are configured to be different multi-layer structures so that a reasonable balance of flexibility and hardness of the middle sheath 2 can be achieved.

Optionally, the fifth outer layer 224 and the fourth outer layer 223 are made of different materials such that a coefficient of friction of the material of the fifth outer layer 224 is less than a coefficient of friction of the material of the fourth outer layer 223, thereby facilitating a nested arrangement and relative sliding of the middle sheath 2 and the outer sheath 1.

In an embodiment, the second wire lumen 24 penetrates through two ends of the middle sheath proximal end 21 along an axial direction of the middle sheath 2 and is disposed in the third outer layer 212 of the middle sheath proximal end 21. In addition, the second wire lumen 24 continues to extend in the fifth outer layer 224 of the middle sheath distal end 22 after penetrating through the middle sheath proximal end 21, so as to form a blind hole structure at the middle sheath distal end 22. The first end of the second pull wire is fixed at the bottom of the blind hole structure, and the second end of the second pull wire protrudes out of the middle sheath proximal end 21. The middle sheath distal end 22 can be deflected by the first deflection angle in the fourth direction relative to the middle sheath proximal end 21 by pulling the second pull wire. The fourth direction here may be the same as or different from the first direction, the second direction, and the third direction and may be adjusted according to requirements. Optionally, the material of the second pull wire may be stainless steel, the nickel titanium alloy, the elgiloy alloy, the cobalt chromium alloy, or the like.

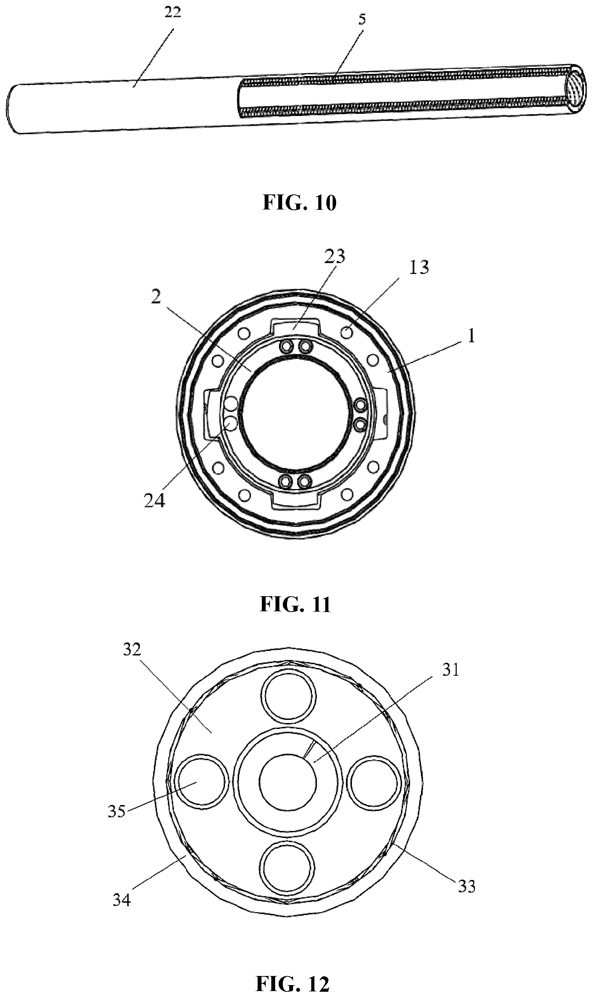

Optionally, as shown in FIG. 10, a reinforcement braided structure 5 is provided inside the second wire lumen 24 at the middle sheath distal end 22, so as to prevent the middle sheath distal end 22 from being too flexible and thus prevent the second pull wire from breaking out of the fifth outer layer 224 of the middle sheath distal end 22 after being deflected to a desired angle, thereby enhancing surgical safety. In this embodiment, the reinforcement braided structure 5 is a spring tube, and the material of the spring tube may be a metal, such as stainless steel and the platinum tungsten alloy, or may be a polymer material, such as PA and HDPE.

As shown in an assembly view of the middle sheath 2 and the outer sheath 1 in FIG. 11, to improve the assembly progress and the accuracy of sliding relative to each other, as shown in FIG. 3, a sliding groove 116 is provided on an inner wall of the first inner layer 111 of the outer sheath proximal end 11, and a slider 23 is provided between the middle sheath proximal end 21 and the middle sheath distal end 22, where the slider 23 is disposed in the sliding groove 116 and can slide along the sliding groove 116. Optionally, the sliding groove 116 is a wedge-shaped groove and the slider 23 is a wedge-shaped block. Multiple sliding grooves 116 are provided. For example, 2 to 8 sliding grooves 116 are provided. Optionally, 2 to 4 sliding grooves 116 are provided. The multiple sliding grooves 116 are uniformly distributed along a circumferential direction of the outer sheath 1. Correspondingly, the number of sliders 23 is the same as the number of the sliding grooves 116, and the sliders 23 and the sliding grooves 116 are slidably connected in a one-to-one correspondence. Optionally, the material of the slider 23 may be a metal, such as stainless steel, the aluminum alloy, and the cobalt chromium alloy, or may be a polymer material, such as PTFE, PA, PEBAX, TPU, PE, or a blend thereof.

Optionally, as shown in FIG. 11, when the outer sheath 1 and the middle sheath 2 are used in combination, the first wire lumen 13 and the second wire lumen 24 are arranged in a staggered manner so that multiple wire lumens can be uniformly distributed at 360° in a certain plane, the first pull wire and the second pull wire penetrate through the first wire lumen 13 and the second wire lumen 24, respectively, so as to deflect the outer sheath 1 and the middle sheath 2, and the omni-directional accurate deflection in a 360° three-dimensional space can be performed. Optionally, the sheath assembly has a spatial deflection angle of -90° to 135°, such as an optional spatial deflection angle of -45° to 90°. The outer sheath 1 and the middle sheath 2 after mating can perform flexible multi-angle accurate conversion, so as to satisfy requirements of the delivery of the mitral valve repair device for the multi-angle conversion. In actual use, the pull wire may be disposed only in the wire lumen in a direction in which an angle needs to be adjusted, and the pull wire may not be disposed in the wire lumen in a direction in which an angle does not need to be adjusted.

As shown in FIG. 12, the inner sheath 3 includes a reinforcement layer 31, a connecting layer 32, a fifth braided layer 33, and a sixth outer layer 34 that are nested from inside to outside in sequence. The sixth inner layer 34 and the connecting layer 32 may both be made of medical polymer materials such as PTFE, PA, PEBAX, TPU, PE, or a blend thereof. The fifth braided layer 33 may be a multi-strand braided structure. Optionally, 16 to 32 braided strands may be provided. In addition to the multi-strand braided structure, the fifth braided layer 33 may be a single-strand or multi-strand spring braided structure, and a braided wire may be a metal flat wire or a metal round wire with a diameter of 0.02 mm to 0.4 mm. The reinforcement layer 31 is a corrugated tube formed by winding a spring, the braided wire is a flat wire or a round wire of 0.3 mm to 1.4 mm, a gap between two adjacent braided wires is not greater than 0.15 mm, and the spring of the reinforcement layer 31 is generally made of stainless steel, the nickel titanium alloy, or the platinum tungsten alloy. Optionally, a gap may exist between the connecting layer 32 and the reinforcement layer 31, or the connecting layer 32 may join the fifth braided layer 33 and the reinforcement layer 31 together without a gap between the connecting layer 32 and the reinforcement layer 31, where in either case, no relative motion occurs between the reinforcement layer 31 and the connecting layer 32.

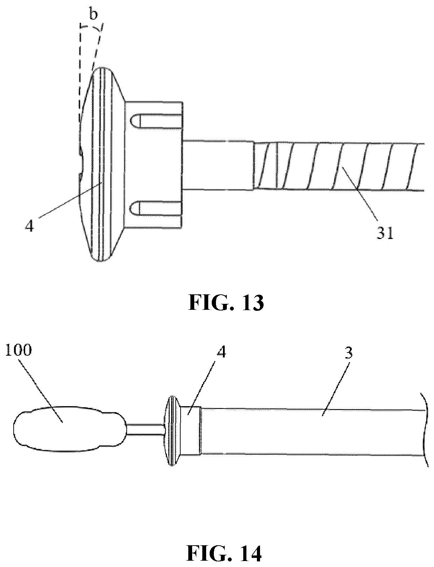

As shown in FIG. 13, a metal fixed head 4 is fixedly connected to a distal end of the inner sheath 3. Compared with a delivery sheath with a soft distal end and a hard proximal end in the existing delivery system, in the delivery system provided in this embodiment, the metal fixed head 4 is provided at the distal end of the inner sheath 3 so that the hardness of the distal end of the entire sheath assembly can be effectively improved, and a hardness variation trend of the entire sheath assembly is approximately hard at the proximal hard-soft in the middle-hard at the distal end. In this manner, during the mitral valve repair surgery, the sheath assembly can effectively resist hypertension in the left ventricle, thereby avoiding the displacement phenomenon of the traditional delivery sheath caused by a pressure difference between the left ventricle and the left atrium and reducing the operation difficulty of the doctor. In this embodiment, the metal fixed head 4 is fixedly connected to the reinforcement layer 31 in the inner sheath 3 in such a manner as to be welded, screwed, or riveted. The metal fixed head 4 is fixedly connected to the reinforcement layer 31 in the inner sheath 3 so that the hardness of the distal end of the sheath assembly is improved, avoiding the displacement phenomenon.

The material of the metal fixed head 4 may be the platinum tungsten alloy, stainless steel, the nickel titanium alloy, the cobalt chromium alloy, or the elgiloy alloy. Optionally, the metal fixed head 4 may interface with both the reinforcement layer 31 and the fifth braided layer 33 at the same time, so as to limit the relative motion between the reinforcement layer 31 and the connecting layer 32 using the metal fixed head 4.

Optionally, a centrally symmetrical groove is provided on a side surface of the metal fixed head 4, where the groove can increase a bonding force between the metal fixed head 4 and the polymer material on the sixth outer layer 34 of the inner sheath 3 and reduce the risk of separation of the sixth outer layer 34 from the metal fixed head 4.

In an embodiment, a lead-in chamfer 41 is circumferentially provided at an end of the metal fixed head 4 facing away from the distal end of the inner sheath 3, and the lead-in chamfer 41 is at a preset acute angle b to an end surface of the metal fixed head 4. Optionally, the preset acute angle b is 5° to 60°. Optionally, the preset acute angle b is 10° to 45°. The lead-in chamber 41 is provided and can effectively reduce the impact of the blood flowing from the left ventricle into the left atrium on the inner sheath 3, so as to effectively resist the hypertension in the left ventricle, thereby avoiding the displacement phenomenon of the traditional delivery sheath caused by a pressure difference between the left ventricle and the left atrium and reducing the operation difficulty of the doctor.

As shown in FIG. 14, the mitral valve repair device (that is, the device to be delivered 100) is fixedly connected to the metal fixed head 4 through a delivery rod in such a manner as to be screwed or riveted.

The present application provides a delivery system for mitral valve repair. The delivery system includes the inner sheath 3, the middle sheath 2, the outer sheath 1, the first pull wire, the second pull wire, and the push-pull wire. The inner sheath 3, the middle sheath 2, and the outer sheath 1 are nested from inside to outside in sequence and slidably connected in sequence along the axial direction of the inner sheath 3. In the delivery system, the first pull wire is used to pull the outer sheath 1 and the second pull wire is used to pull the middle sheath 2 so that the outer sheath distal end 12 and the middle sheath distal end 22 can be deflected by different angles in different directions, thereby increasing the flexibility of the delivery system when delivering the device to be delivered 100.

In addition, in the delivery system, the hardness of the outer sheath proximal end 11 is set to be greater than the hardness of the outer sheath distal end 12, the hardness of the middle sheath proximal end 21 is set to be greater than the hardness of the middle sheath distal end 22, and the outer sheath 1 and the middle sheath 2 are pre-formed so that the outer sheath distal end 12 is deflected by the first predetermined angle a in the first direction relative to the outer sheath proximal end 11, and the middle sheath distal end 22 is deflected by the second predetermined angle in the second direction relative to the middle sheath proximal end 21, thereby reducing the operation difficulty of the delivery system and reducing dependence on the technical level and clinical experience of the doctor.

## Claims

1. A delivery system for mitral valve repair, comprising:
a sheath assembly comprising an outer sheath (1), a middle sheath (2), and an inner sheath (3), wherein the inner sheath (3), the middle sheath (2), and the outer sheath (1) are nested from inside to outside in sequence and slidably connected in sequence along an axial direction of the inner sheath (3), the outer sheath (1) comprises an outer sheath proximal end (11) and an outer sheath distal end (12) which are connected to each other, hardness of the outer sheath proximal end (11) is greater than hardness of the outer sheath distal end (12), the middle sheath (2) comprises a middle sheath proximal end (21) and a middle sheath distal end (22) which are connected to each other, hardness of the middle sheath proximal end (21) is greater than hardness of the middle sheath distal end (22)

2. The delivery system for mitral valve repair of claim 1, wherein
the outer sheath proximal end (11) comprises a first inner layer (111), a first braided layer (112), a reinforcement layer (113), a second braided layer (114), and a first outer layer (115), wherein the first inner layer (111), the first braided layer (112), the reinforcement layer (113), the second braided layer (114), and the first outer layer (115) are nested from inside to outside in sequence; and
the outer sheath distal end (12) comprises a second inner layer (121), a third braided layer (122), and a second outer layer (123), wherein the second inner layer (121), the third braided layer (122), and the second outer layer (123) are nested from inside to outside in sequence.

3. The delivery system for mitral valve repair of claim 2, further comprising at least one of the following features:
a braid density of the first braided layer (112) is greater than a braid density of the second braided layer (114); and
a braid density of the third braided layer (122) is less than a braid density of the second braided layer (114).

4. The delivery system for mitral valve repair of claim 2, further comprising:
a first pull wire, wherein the outer sheath (1) is provided with a first wire lumen (13) along an axial direction of the outer sheath (1), the first pull wire is disposed in the first wire lumen (13), a first end of the first pull wire is fixedly connected to the outer sheath distal end (12), and a second end of the first pull wire is configured to deflect the outer sheath distal end (12) by a first deflection angle relative to the outer sheath proximal end (11) when the second end of the first pull wire is pulled;
wherein the first wire lumen (13) penetrates through two ends of the outer sheath proximal end (11) and an end of the second outer layer (123) facing the outer sheath proximal end (11).

5. The delivery system for mitral valve repair of claim 4, wherein
a reinforcement braided structure (5) is provided in part of the first wire lumen (13) located at the outer sheath distal end (12).

6. The delivery system for mitral valve repair of claim 1, wherein
the middle sheath proximal end (21) comprises a third inner layer (211) and a third outer layer (212) which are nested from inside to outside in sequence; and
the middle sheath distal end (22) comprises a fourth inner layer (221), a fourth braided layer (222), a fourth outer layer (223), and a fifth outer layer (224), wherein the fourth inner layer (221), the fourth braided layer (222), the fourth outer layer (223), and the fifth outer layer (224) are nested from inside to outside in sequence.

7. The delivery system for mitral valve repair of claim 5, further comprising:
a second pull wire, wherein the middle sheath (2) is provided with a second wire lumen (24) along an axial direction of the middle sheath (2), the second pull wire is disposed in the second wire lumen (24), a first end of the second pull wire is fixedly connected to the middle sheath distal end (22), and a second end of the second pull wire is configured to deflect the middle sheath distal end (22) by a second deflection angle relative to the middle sheath proximal end (21) when the second end of the second pull wire is pulled;
wherein the second wire lumen (24) penetrates through two ends of the third outer layer (212) and an end of the fourth outer layer (223) facing the middle sheath proximal end (21).

8. The delivery system for mitral valve repair of claim 7, wherein
a reinforcement braided structure (5) is provided in part of the second wire lumen (24) located at the middle sheath distal end (22).

9. The delivery system for mitral valve repair of claim 1, wherein
the inner sheath (3) comprises a reinforcement layer (31), a connecting layer (32), a fifth braided layer (33), and a sixth outer layer (34), wherein the reinforcement layer (31), the connecting layer (32), the fifth braided layer (33), and the sixth outer layer (34) are nested from inside to outside in sequence.

10. The delivery system for mitral valve repair of claim 9, further comprising:
a push-pull wire, wherein a third wire lumen (35) penetrates through two ends of the connecting layer (32) along the axial direction of the inner sheath (3), the push-pull wire is disposed in the third wire lumen (35), a first end of the push-pull wire is connected to a device to be delivered (100), and a second end of the push-pull wire is configured to be capable of adjusting a position of the device to be delivered (100) when the second end of the push-pull wire is pushed or pulled.

11. The delivery system for mitral valve repair of claim 1, wherein
a sliding groove (116) is provided on one of an inner wall of the outer sheath (1) and an outer wall of the middle sheath (2), and a slider (23) is provided on an other one of the inner wall of the outer sheath (1) and the outer wall of the middle sheath (2), wherein the slider (23) is slidably connected in the sliding groove (116).

12. The delivery system for mitral valve repair of claim 1, further comprising:
a metal fixed head (4) fixedly connected to a distal end of the inner sheath (3).

13. The delivery system for mitral valve repair of claim 12, wherein
a lead-in chamfer (41) is circumferentially provided at an end of the metal fixed head (4) facing away from the distal end of the inner sheath (3), and the lead-in chamfer (41) is at a preset acute angle to an end surface of the metal fixed head (4).

14. The delivery system for mitral valve repair of claim 1, further comprising at least one of the following features:
a plurality of first wire lumens (13) are provided, the plurality of first wire lumens (13) are uniformly distributed along a circumferential direction of the outer sheath (1), and at least one first pull wire is provided in each of the plurality of first wire lumens (13);
a plurality of second wire lumens (24) are provided, the plurality of second wire lumens (24) are uniformly distributed along a circumferential direction of the middle sheath (2), and at least one second pull wire is provided in each of the plurality of second wire lumens (24); and
a plurality of third wire lumens (35) are provided, the plurality of third wire lumens (35) are uniformly distributed along a circumferential direction of the inner sheath (3), and at least one push-pull wire is provided in each of the plurality of third wire lumens (35).

15. The delivery system for mitral valve repair of claim 1, wherein the outer sheath distal end (12) is capable of being deflected by a first predetermined angle (a) in a first direction relative to the outer sheath proximal end (11), and the middle sheath distal end (22) is capable of being deflected by a second predetermined angle in a second direction relative to the middle sheath proximal end (21).

## Patentansprüche

1. Einführsystem für Mitralklappenreparatur, umfassend:
eine Hüllenanordnung, umfassend eine äußere Hülle (1), eine mittlere Hülle (2) und eine innere Hülle (3), wobei die innere Hülle (3), die mittlere Hülle (2) und die äußere Hülle (1) nacheinander von innen nach außen eingepasst und verschiebbar nacheinander entlang einer axialen Richtung der inneren Hülle (3) verbunden sind, die äußere Hülle (1) ein proximales Ende (11) der äußeren Hülle und ein distales Ende (12) der äußeren Hülle, die miteinander verbunden sind, umfasst, die Härte des proximalen Endes (11) der äußeren Hülle größer ist als die Härte des distalen Endes (12) der äußeren Hülle, die mittlere Hülle (2) ein proximales Ende (21) der mittleren Hülle und ein distales Ende (22) der mittleren Hülle umfasst, die miteinander verbunden sind, und die Härte des proximalen Endes (21) der mittleren Hülle größer ist als die Härte des distalen Endes (22) der mittleren Hülle.

2. Einführsystem für Mitralklappenreparatur nach Anspruch 1, wobei
das proximale Ende (11) der äußeren Hülle eine erste innere Schicht (111), eine erste geflochtene Schicht (112), eine Verstärkungsschicht (113), eine zweite geflochtene Schicht (114) und eine erste äußere Schicht (115) umfasst, wobei die erste innere Schicht (111), die erste geflochtene Schicht (112), die Verstärkungsschicht (113), die zweite geflochtene Schicht (114) und die erste äußere Schicht (115) nacheinander von innen nach außen eingepasst sind und
das distale Ende (12) der äußeren Hülle eine zweite innere Schicht (121), eine dritte geflochtene Schicht (122) und eine zweite äußere Schicht (123) umfasst, wobei die zweite innere Schicht (121), die dritte geflochtene Schicht (122) und die zweite äußere Schicht (123) nacheinander von innen nach außen eingepasst sind.

3. Einführsystem für Mitralklappenreparatur nach Anspruch 2, ferner umfassend mindestens eins der folgenden Merkmale:
eine Geflechtdichte der ersten geflochtenen Schicht (112) ist größer als eine Geflechtdichte der zweiten geflochtenen Schicht (114) und
eine Geflechtdichte der dritten geflochtenen Schicht (122) ist kleiner als eine Geflechtdichte der zweiten geflochtenen Schicht (114).

4. Einführsystem für Mitralklappenreparatur nach Anspruch 2, ferner Folgendes umfassend:
einen ersten Zugdraht, wobei die äußere Hülle (1) mit einem ersten Drahtlumen (13) entlang einer axialen Richtung der äußeren Hülle (1) versehen ist, der erste Zugdraht in dem ersten Drahtlumen (13) angeordnet ist, ein erstes Ende des ersten Zugdrahts fest mit dem distalen Ende (12) der äußeren Hülle verbunden ist und ein zweites Ende des ersten Zugdrahts ausgelegt ist, um das distale Ende (12) der äußeren Hülle um einen ersten Ablenkwinkel relativ zum proximalen Ende (11) der äußeren Hülle abzulenken, wenn das zweite Ende des ersten Zugdrahts gezogen wird;
wobei das erste Drahtlumen (13) durch zwei Enden des proximalen Endes (11) der äußeren Hülle und ein Ende der zweiten äußeren Schicht (123) dringt, das dem proximalen Ende (11) der äußeren Hülle zugewandt ist.

5. Einführsystem für Mitralklappenreparatur nach Anspruch 4, wobei
eine geflochtene Verstärkungsstruktur (5) in einem Teil des ersten Drahtlumens (13) befindlich an dem distalen Ende (12) der äußeren Hülle bereitgestellt ist.

6. Einführsystem für Mitralklappenreparatur nach Anspruch 1, wobei
das proximale Ende (21) der mittleren Hülle eine dritte innere Schicht (211) und eine dritte äußere Schicht (212) umfasst, die nacheinander von innen nach außen eingepasst sind, und
das distale Ende (22) der mittleren Hülle eine vierte innere Schicht (221), eine vierte geflochtene Schicht (222), eine vierte äußere Schicht (223) und eine fünfte äußere Schicht (224) umfasst, wobei die vierte innere Schicht (221), die vierte geflochtene Schicht (222), die vierte äußere Schicht (223) und die fünfte äußere Schicht (224) nacheinander von innen nach außen eingepasst sind.

7. Einführsystem für Mitralklappenreparatur nach Anspruch 5, ferner Folgendes umfassend:
einen zweiten Zugdraht, wobei die mittlere Hülle (2) mit einem zweiten Drahtlumen (24) entlang einer axialen Richtung der mittleren Hülle (2) versehen ist, der zweite Zugdraht in dem zweiten Drahtlumen (24) angeordnet ist, ein erstes Ende des zweiten Zugdrahts fest mit dem distalen Ende (22) der mittleren Hülle verbunden ist und ein zweites Ende des zweiten Zugdrahts ausgelegt ist, um das distale Ende (22) der mittleren Hülle um einen zweiten Ablenkwinkel relativ zum proximalen Ende (21) der mittleren Hülle abzulenken, wenn das zweite Ende des zweiten Zugdrahts gezogen wird;
wobei das zweite Drahtlumen (24) durch zwei Enden der dritten äußeren Schicht (212) und ein Ende der vierten äußeren Schicht (223) dringt, das dem proximalen Ende (21) der mittleren Hülle zugewandt ist.

8. Einführsystem für Mitralklappenreparatur nach Anspruch 7, wobei
eine geflochtene Verstärkungsstruktur (5) in einem Teil des zweiten Drahtlumens (24) befindlich an dem distalen Ende (22) der mittleren Hülle bereitgestellt ist.

9. Einführsystem für Mitralklappenreparatur nach Anspruch 1, wobei
die innere Hülle (3) eine Verstärkungsschicht (31), eine Verbindungsschicht (32), eine fünfte geflochtene Schicht (33) und eine sechste äußere Schicht (34) umfasst, wobei die Verstärkungsschicht (31), die Verbindungsschicht (32), die fünfte geflochtene Schicht (33) und die sechste äußere Schicht (34) nacheinander von innen nach außen eingepasst sind.

10. Einführsystem für Mitralklappenreparatur nach Anspruch 9, ferner Folgendes umfassend:
einen Schiebe-Zug-Draht, wobei ein drittes Drahtlumen (35) durch zwei Enden der Verbindungsschicht (32) entlang der axialen Richtung der inneren Hülle (3) dringt, der Schiebe-Zug-Draht im dritten Drahtlumen (35) angeordnet ist, ein erstes Ende des Schiebe-Zug-Drahts mit einer einzuführenden Vorrichtung (100) verbunden ist und ein zweites Ende des Schiebe-Zug-Drahts ausgelegt ist, um in der Lage zu sein, eine Position der einzuführenden Vorrichtung (100) zu regeln, wenn das zweite Ende des Schiebe-Zug-Drahts geschoben oder gezogen wird.

11. Einführsystem für Mitralklappenreparatur nach Anspruch 1, wobei
eine Schiebenut (116) entweder an einer inneren Wand der äußeren Hülle (1) oder an einer äußeren Wand der mittleren Hülle (2) bereitgestellt ist und ein Schieber (23) auf einem anderen der beiden Elemente, also entweder der inneren Wand der äußeren Hülle (1) oder der äußeren Wand der mittleren Hülle (2) bereitgestellt ist, wobei der Schieber (23) verschiebbar in der Schiebenut (116) verbunden ist.

12. Einführsystem für Mitralklappenreparatur nach Anspruch 1, ferner Folgendes umfassend:
einen festen Metallkopf (4), der fest mit einem distalen Ende der inneren Hülle (3) verbunden ist.

13. Einführsystem für Mitralklappenreparatur nach Anspruch 12, wobei
eine Einführfase (41) umfangseitig an einem Ende des festen Metallkopfs (4), das von dem distalen Ende der inneren Hülle (3) abgewandt ist, bereitgestellt ist und die Einführfase (41) sich an einem voreingestellten spitzen Winkel zu einer Endoberfläche des festen Metallkopfs (4) befindet.

14. Einführsystem für Mitralklappenreparatur nach Anspruch 1, ferner umfassend mindestens eins der folgenden Merkmale:
eine Vielzahl von ersten Drahtlumen (13) ist bereitgestellt, die Vielzahl von ersten Drahtlumen (13) ist gleichmäßig entlang einer Umfangsrichtung der äußeren Hülle (1) verteilt und mindestens ein erster Zugdraht ist in einem jeden der Vielzahl von ersten Drahtlumen (13) bereitgestellt;
eine Vielzahl von zweiten Drahtlumen (24) ist bereitgestellt, die Vielzahl von zweiten Drahtlumen (24) ist gleichmäßig entlang einer Umfangsrichtung der mittleren Hülle (2) verteilt und mindestens ein zweiter Zugdraht ist in einem jeden der Vielzahl von zweiten Drahtlumen (24) bereitgestellt und
eine Vielzahl von dritten Drahtlumen (35) ist bereitgestellt, die Vielzahl von dritten Drahtlumen (35) ist gleichmäßig entlang einer Umfangsrichtung der inneren Hülle (3) verteilt und mindestens ein Schiebe-Zug-Draht ist in einem jeden der Vielzahl von dritten Drahtlumen (35) bereitgestellt.

15. Einführsystem für Mitralklappenreparatur nach Anspruch 1, wobei das distale Ende (12) der äußeren Hülle in der Lage ist, um einen ersten vorbestimmten Winkel (a) in eine erste Richtung relativ zu dem proximalen Ende (11) der äußeren Hülle abgelenkt zu werden, und das distale Ende (22) der mittleren Hülle in der Lage ist, um einen zweiten vorbestimmten Winkel in eine zweite Richtung relativ zu dem proximalen Ende (21) der mittleren Hülle abgelenkt zu werden.

## Revendications

1. Système d'administration pour la réparation de valvule mitrale, comprenant :
un assemblage de gaines comprenant une gaine externe (1), une gaine intermédiaire (2) et une gaine interne (3), dans lequel la gaine interne (3), la gaine intermédiaire (2) et la gaine externe (1) sont imbriquées de l'intérieur vers l'extérieur en séquence et reliées de manière coulissante en séquence le long d'une direction axiale de la gaine interne (3), la gaine externe (1) comprend une extrémité proximale de gaine externe (11) et une extrémité distale de gaine externe (12) qui sont reliées l'une à l'autre, la dureté de l'extrémité proximale de gaine externe (11) est supérieure à la dureté de l'extrémité distale de gaine externe (12), la gaine intermédiaire (2) comprend une extrémité proximale de gaine intermédiaire (21) et une extrémité distale de gaine intermédiaire (22) qui sont reliées l'une à l'autre, la dureté de l'extrémité proximale de gaine intermédiaire (21) est supérieure à la dureté de l'extrémité distale de gaine intermédiaire (22).

2. Système d'administration pour la réparation de valvule mitrale selon la revendication 1, dans lequel
l'extrémité proximale de gaine externe (11) comprend une première couche interne (111), une première couche tressée (112), une couche de renfort (113), une deuxième couche tressée (114), et une première couche externe (115), dans lequel la première couche interne (111), la première couche tressée (112), la couche de renfort (113), la deuxième couche tressée (114) et la première couche externe (115) sont imbriquées de l'intérieur vers l'extérieur en séquence ; et
l'extrémité distale de gaine externe (12) comprend une deuxième couche interne (121), une troisième couche tressée (122) et une deuxième couche externe (123), dans lequel la deuxième couche interne (121), la troisième couche tressée (122) et la deuxième couche externe (123) sont imbriquées de l'intérieur vers l'extérieur en séquence.

3. Système d'administration pour la réparation de valvule mitrale selon la revendication 2, comprenant en outre au moins l'une des caractéristiques suivantes :
une densité de tresse de la première couche tressée (112) est supérieure à une densité de tresse de la deuxième couche tressée (114) ; et
une densité de tresse de la troisième couche tressée (122) est inférieure à une densité de tresse de la deuxième couche tressée (114).

4. Système d'administration pour la réparation de valvule mitrale selon la revendication 2, comprenant en outre :
un premier fil de traction, dans lequel la gaine externe (1) est pourvue d'une première lumière de fil (13) le long d'une direction axiale de la gaine externe (1), le premier fil de traction est disposé dans la première lumière de fil (13), une première extrémité du premier fil de traction est reliée de manière fixe à l'extrémité distale de gaine externe (12), et une deuxième extrémité du premier fil de traction est configurée pour dévier l'extrémité distale de gaine externe (12) d'un premier angle de déviation par rapport à l'extrémité proximale de gaine externe (11) lorsque la deuxième extrémité du premier fil de traction est tirée ;
dans lequel la première lumière de fil (13) traverse les deux extrémités de l'extrémité proximale de gaine externe (11) et une extrémité de la deuxième couche externe (123) faisant face à l'extrémité proximale de gaine externe (11).

5. Système d'administration pour la réparation de valvule mitrale selon la revendication 4, dans lequel
une structure tressée de renfort (5) est ménagée dans une partie de la première lumière de fil (13) située au niveau de l'extrémité distale de gaine externe (12).

6. Système d'administration pour la réparation de valvule mitrale selon la revendication 1, dans lequel
l'extrémité proximale de gaine intermédiaire (21) comprend une troisième couche interne (211) et une troisième couche externe (212) qui sont imbriquées de l'intérieur vers l'extérieur en séquence ; et
l'extrémité distale de gaine intermédiaire (22) comprend une quatrième couche interne (221), une quatrième couche tressée (222), une quatrième couche externe (223) et une cinquième couche externe (224), dans lequel la quatrième couche interne (221), la quatrième couche tressée (222), la quatrième couche externe (223) et la cinquième couche externe (224) sont imbriquées de l'intérieur vers l'extérieur en séquence.

7. Système d'administration pour la réparation de valvule mitrale selon la revendication 5, comprenant en outre :
un deuxième fil de traction, dans lequel la gaine intermédiaire (2) est pourvue d'une deuxième lumière de fil (24) le long d'une direction axiale de la gaine intermédiaire (2), le deuxième fil de traction est disposé dans la deuxième lumière de fil (24), une première extrémité du deuxième fil de traction est reliée de manière fixe à l'extrémité distale de gaine intermédiaire (22), et une deuxième extrémité du deuxième fil de traction est configurée pour dévier l'extrémité distale de gaine intermédiaire (22) d'un deuxième angle de déviation par rapport à l'extrémité proximale de gaine intermédiaire (21) lorsque la deuxième extrémité du deuxième fil de traction est tirée ;
dans lequel la deuxième lumière de fil (24) traverse les deux extrémités de la troisième couche externe (212) et une extrémité de la quatrième couche externe (223) faisant face à l'extrémité proximale de gaine intermédiaire (21).

8. Système d'administration pour la réparation de valvule mitrale selon la revendication 7, dans lequel
une structure tressée de renfort (5) est ménagée dans une partie de la deuxième lumière de fil (24) située au niveau de l'extrémité distale de gaine intermédiaire (22).

9. Système d'administration pour la réparation de valvule mitrale selon la revendication 1, dans lequel
la gaine interne (3) comprend une couche de renfort (31), une couche de liaison (32), une cinquième couche tressée (33) et une sixième couche externe (34), dans lequel la couche de renfort (31), la couche de liaison (32), la cinquième couche tressée (33) et la sixième couche externe (34) sont imbriquées de l'intérieur vers l'extérieur en séquence.

10. Système d'administration pour la réparation de valvule mitrale selon la revendication 9, comprenant en outre :
un fil de poussée-traction, dans lequel une troisième lumière de fil (35) traverse les deux extrémités de la couche de liaison (32) le long de la direction axiale de la gaine interne (3), le fil de poussée-traction est disposé dans la troisième lumière de fil (35), une première extrémité du fil de poussée-traction est reliée à un dispositif à administrer (100), et une deuxième extrémité du fil de poussée-traction est configurée pour ajuster une position du dispositif à administrer (100) lorsque la deuxième extrémité du fil de poussée-traction est poussée ou tirée.

11. Système d'administration pour la réparation de valvule mitrale selon la revendication 1, dans lequel
une rainure de coulissement (116) est ménagée sur l'une parmi une paroi interne de la gaine externe (1) et une paroi externe de la gaine intermédiaire (2), et un coulisseau (23) est ménagé sur une autre parmi la paroi interne de la gaine externe (1) et la paroi externe de la gaine intermédiaire (2), dans lequel le coulisseau (23) est raccordé de manière coulissante dans la rainure de coulissement (116).

12. Système d'administration pour la réparation de valvule mitrale selon la revendication 1, comprenant en outre :
une tête fixe métallique (4) reliée de manière fixe à une extrémité distale de la gaine interne (3).

13. Système d'administration pour la réparation de valvule mitrale selon la revendication 12, dans lequel
un biseau d'entrée (41) est ménagé circonférentiellement au niveau d'une extrémité de la tête fixe métallique (4) opposée à l'extrémité distale de la gaine interne (3), et le biseau d'entrée (41) est à un angle aigu prédéfini par rapport à une surface d'extrémité de la tête fixe métallique (4).

14. Système d'administration pour la réparation de valvule mitrale selon la revendication 1, comprenant en outre au moins l'une des caractéristiques suivantes :
une pluralité de premières lumières de fil (13) est ménagée, la pluralité de premières lumières de fil (13) est répartie uniformément le long d'une direction circonférentielle de la gaine externe (1), et au moins un premier fil de traction est ménagé dans chacune de la pluralité de premières lumières de fil (13) ;
une pluralité de deuxièmes lumières de fil (24) est ménagée, la pluralité de deuxièmes lumières de fil (24) est répartie uniformément le long d'une direction circonférentielle de la gaine intermédiaire (2), et au moins un deuxième fil de traction est ménagé dans chacune de la pluralité de deuxièmes lumières de fil (24) ; et
une pluralité de troisièmes lumières de fil (35) est ménagée, la pluralité de troisièmes lumières de fil (35) est répartie uniformément le long d'une direction circonférentielle de la gaine interne (3), et au moins un fil de poussée-traction est ménagé dans chacune de la pluralité de troisièmes lumières de fil (35).

15. Système d'administration pour la réparation de valvule mitrale selon la revendication 1, dans lequel l'extrémité distale de gaine externe (12) est capable d'être déviée d'un premier angle prédéterminé (a) dans une première direction par rapport à l'extrémité proximale de gaine externe (11), et l'extrémité distale de gaine intermédiaire (22) est capable d'être déviée d'un deuxième angle prédéterminé dans une deuxième direction par rapport à l'extrémité proximale de gaine intermédiaire (21).
